# EUROPÄISCHE PATENTSCHRIFT

(11) **EP 1 763 314 B1**
(45) Veröffentlichungstag und Bekanntmachung des Hinweises auf die Patenterteilung: **17.08.2011**
(21) Anmeldenummer: 05755658.1
(22) Anmeldetag: 09.06.2005
(51) Int. Cl.: A61B 1/015

(54) **SPÜLEINRICHTUNG UND VERFAHREN ZUM BETRIEB EINER SPÜLEINRICHTUNG**
RINSING DEVICE AND METHOD FOR THE OPERATION THEREOF
DISPOSITIF DE NETTOYAGE ET PROCEDE POUR FAIRE FONCTIONNER UN DISPOSITIF DE NETTOYAGE

(30) Priorität: 11.06.2004 DE 102004028361
(43) Veröffentlichungstag der Anmeldung: 21.03.2007
(73) Patentinhaber: Erbe Elektromedizin GmbH, 72072 Tübingen (DE)
(72) Erfinder: BAUR, Thomas, 72108 Rottenburg (DE)
(74) Vertreter: Bohnenberger, Johannes
(86) Internationale Anmeldenummer: PCT/EP2005/006201
(87) Internationale Veröffentlichungsnummer: WO 2005/120328

(56) Entgegenhaltungen:
- DE-C1- 19 525 926
- US-A- 4 998 914
- US-A1- 2001 039 370
- US-A1- 2004 097 872

## Beschreibung

Die Erfindung betrifft eine Spüleinrichtung insbesondere für die Endoskopie sowie ein Verfahren zum Betrieb einer derartigen Spüleinrichtung.

Spüleinrichtungen, die für medizinische Anwendungen verwendet werden, umfassen üblicherweise eine Peristaltik-Rollenpumpe, mittels derer ohne die Gefahr von Kontamination ein Spül-Fluid (z.B. Ringerlösung) einem Applikator zugeführt wird, um einen Spülvorgang z.B. in einem Operationsbereich durchzuführen. In der Endoskopie werden derartige Spüleinrichtungen darüber hinaus verwendet, um die optischen Einrichtungen des Endoskops zu reinigen.

Eine Problematik bei derartigen Spüleinrichtungen besteht darin, dass Stenosen z.B. durch Verstopfung oder Abknicken des Schlauches auftreten können, welche zu einem Platzen des Schlauches führen können. Dies bedeutet eine Gefährdung des Patienten, die unbedingt zu vermeiden ist.

Weiterhin werden derartige Spüleinrichtungen je nach Einsatzort mit verschiedenen Applikatoren verwendet, was insbesondere beim Einsatz von dünnen Spülsonden und einer von der Bedienungsperson zu hoch eingestellten Fördermenge ebenfalls zum Platzen des Schlauches führen kann.

Eine Spüleinrichtung nach dem Oberbegiff des Ansprüchs 1 ist aus der US 2004/0097872A1 bekannt.

Der Erfindung liegt die Aufgabe zu Grunde, eine Spüleinrichtung der eingangs genannten Art dahin gehend weiter zu bilden, dass ein sicherer Betrieb der Spüleinrichtung selbsttätig einstellbar ist. Weiterhin ist es Aufgabe der Erfindung, ein Verfahren zum Betrieb einer Spüleinrichtung aufzuzeigen, welches die beschriebenen Probleme löst.

Vorrichtungsmäßig wird die Aufgabe durch eine Spüleinrichtung insbesondere für die Endoskopie gelöst, die eine elektrisch angetriebene Pumpe, insbesondere eine PeristaltikRollenpumpe und eine Steuerungseinrichtung aufweist zum Feststellen und/oder Einstellen einer Durchflussmenge, wobei eine Strom-Messeinrichtung zum Feststellen eines von der Pumpe aufgenommenen Stroms vorgesehen und die Steuerungseinrichtung derart ausgebildet ist, dass dann, wenn die Durchflussmenge einen voreingestellten Durchfluss-Grenzwert überschreitet und gleichzeitig der Strom einen ersten voreingestellten Stromwert überschreitet, die Durchflussmenge auf einen Durchfluss-Sicherheitswert reduziert wird.

Ein wesentlicher Punkt der Erfindung liegt darin, dass ohne die gesonderte Messung des Spüldruckes nur aufgrund der Strom-Messung und dessen Vergleich mit einem Schwellenwert bei bekannter Durchflussmenge diese bei ansteigendem Strom auf einen ungefährlichen Wert reduziert wird. Selbst dann, wenn eine Durchflussmenge von der Bedienungsperson eingestellt wurde, welche für eine (von der Bedienungsperson angebrachte) dünne Spülsonde zu hoch wäre, also einen gefährlich hohen Druck erzeugen würde, wird die Gefährdung selbsttätig sicher vermieden. Wenn nämlich der eingestellte Durchfluss unterhalb des Sicherheitswertes liegt, tritt ein Platzen des Schlauches nicht auf.

Vorzugsweise ist die Steuerungseinrichtung derart ausgebildet, dass der Durchfluss-Sicherheitswert gleich dem Durchfluss-Grenzwert oder etwas niedriger ist, um eine Hysterese zu erreichen. In jedem Fall wird aber die Durchflussmenge so weit reduziert, dass keine Gefahr mehr gegeben ist.

Die Steuerungseinrichtung ist vorzugsweise derart ausgebildet, dass dann, wenn der Strom einen zweiten voreingestellten Stromwert überschreitet, der höher ist als der erste Stromwert, die Pumpe (vollständig) abgeschaltet und/oder ein Warnsignal erzeugt wird. Ein solch hoher Stromwert bedeutet nämlich, dass der Motor (z.B. durch Probleme an den Rollen) blockiert ist. Mit dieser Maßnahme wird ein Schutz der Spüleinrichtung sichergestellt.

Wenn die Durchflussmenge in der oben beschriebnen Art auf den Durchfluss-Sicherheitswert einmal reduziert wurde, so wird bei einer bevorzugten Ausführungsform der Erfindung die Durchflussmenge mit einer voreingestellten Steigerungsrate, sozusagen schrittweise bis zu einer Durchflussmenge erhöht, welche der von der Bedienungsperson eingestellten Soll-Durchflussmenge entspricht. Überschreitet der Strom wieder den ersten voreingestellten Stromwert, so wird wieder die Durchflussmenge auf den ungefährlichen Wert reduziert. Bei einer vorübergehenden Stenose des Schlauches, also z.B. bei einem zeitweisen Abdrücken oder Abknicken führt dies somit einerseits nicht zu einem Platzen des Schlauches, andererseits wird aber nach einem Beheben der Stenose wieder der von der Bedienungsperson gewünschte Sollwert erreicht.

Verfahrensmäßig wird die Aufgabe bei einem Verfahren zum Betrieb einer Spüleinrichtung insbesondere für die Endoskopie, umfassend eine elektrisch angetriebene Pumpe, insbesondere Peristaltik-Rollenpumpe, dadurch gelöst, dass eine Durchflussmenge festgestellt und/oder eingestellt wird, ein von der Pumpe aufgenommener Strom festgestellt und die Durchflussmenge dann auf einen Durchfluss-Sicherheitswert reduziert wird, wenn die Durchflussmenge einen voreingestellten Durchfluss-Grenzwert überschreitet und gleichzeitig der Strom einen ersten voreingestellten Stromwert überschreitet. Hierbei ist der Durchfluss-Sicherheitswert gleich dem Durchfluss-Grenzwert.

Weiterhin wird dann, wenn der Strom einen zweiten voreingestellten Stromwert überschreitet, der höher ist als der erste Stromwert, die Pumpe abgeschaltet und/oder ein Warnsignal erzeugt.

Nach einem Reduzieren der Durchflussmenge auf den Durchfluss-Sicherheitswert wird die Durchflussmenge mit einer voreingestellten Steigerungsrate (schrittweise) bis zu einer voreingestellten Soll-Durchflussmenge erhöht.

Bevorzugte Ausführungsformen der Erfindung ergeben sich aus den Unteransprüchen.

Nachfolgend wird ein Ausführungsbeispiel der Erfindung anhand von Abbildungen näher erläutert. Hierbei zeigen
- Fig. 1 ein Blockschaltbild einer Ausführungsform der Spüleinrichtung,
- Fig. 2 ein Flussdiagramm zur Erläuterung der Reduktion der Durchflussmenge und
- Fig. 3 ein Flussdiagramm zur Erläuterung der Erhöhung der Durchflussmenge nach Auftreten eines Problems.

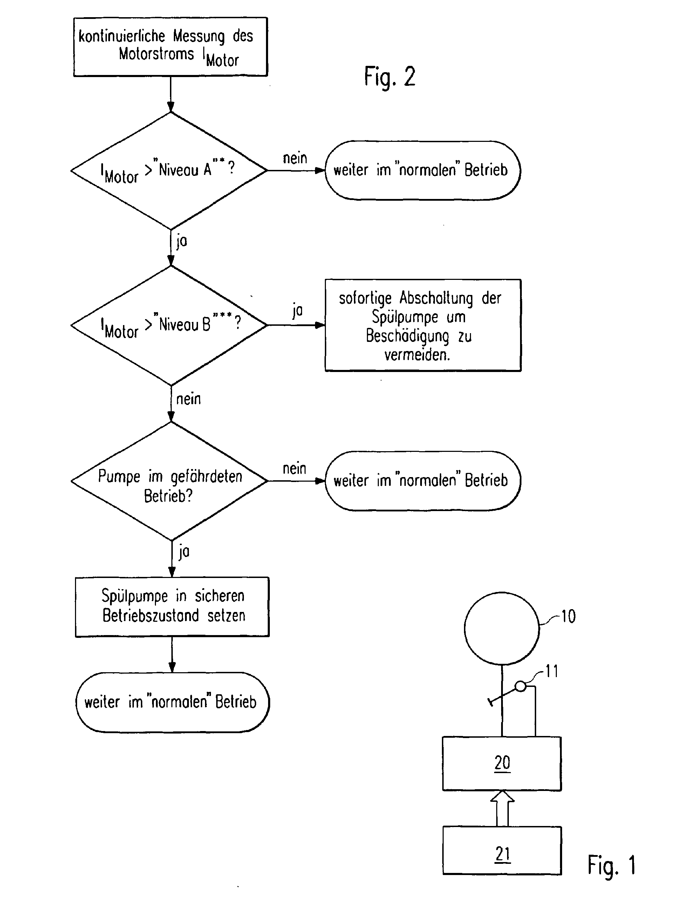

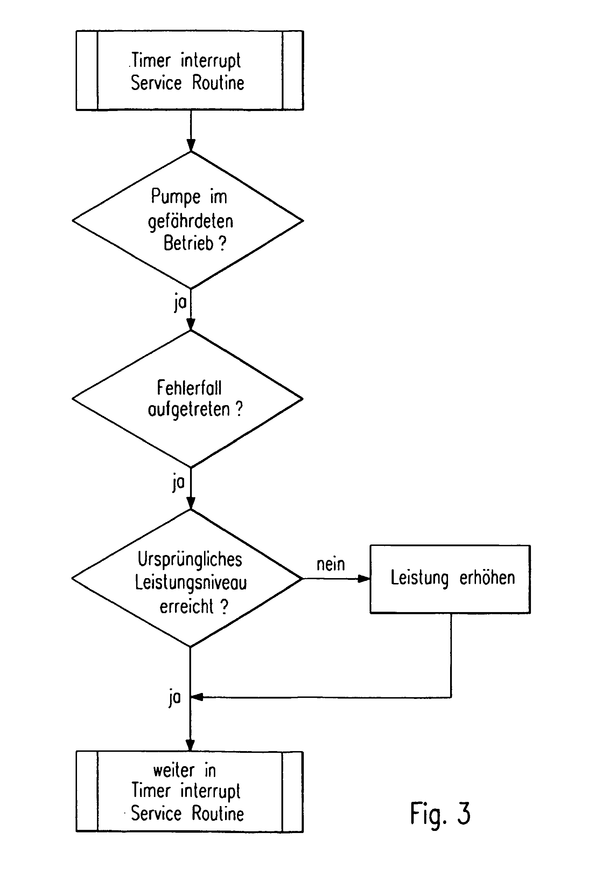

In Fig. 1 ist in stark schematisierter Weise eine Spüleinrichtung gezeigt, welche eine Pumpe 10 mit einem entsprechenden elektrischen Pumpenmotor und eine Steuerungseinrichtung 20 aufweist, welche den Elektromotor der Pumpe treibt. Zur Messung des Stroms ist eine Strom-Messeinrichtung 11 vorgesehen, deren Strom-Messsignal der Steuerungseinrichtung 20 zugeführt wird. Zum Eingeben einer Förderleistung (Durchflussmenge) ist eine Eingabeeinrichtung 21 vorgesehen. Die Steuerungseinrichtung 20 umfasst einen Mikrocomputer sowie Speichereinrichtungen zum Speichern der eingegebenen und der gemessenen Werte sowie zum (permanenten) Speichern von Grenzwerten. Dies wird nachfolgend anhand der Fig. 2 und 3 erläutert.

Der Strom, den der Motor der Pumpe 10 aufnimmt, wird kontinuierlich vom Fühler 11 gemessen und ein entsprechendes Messsignal wird der Steuerungseinrichtung 20 zugeführt. Diese stellt in einem ersten Schritt fest, ob der Strom einen ersten voreingestellten Stromwert (Niveau A) überschreitet. Ist dies nicht der Fall, wird die Pumpe in der üblichen Weise betrieben, also so, dass der Motor zur Erreichung der eingestellten Fördermenge betrieben wird. Wird der erste voreingestellte Stromwert überschritten, so wird in einem zweiten Schritt abgefragt, ob der Strom einen zweiten voreingestellten Stromwert überschreitet, der höher ist als der ersten Stromwert. Ist dies der Fall, so wird der Motor der Spülpumpe vollständig abgeschaltet, um Beschädigungen zu vermeiden. Weiterhin wird ein Warnsignal ausgegeben. Überschreitet der Strom den zweiten voreingestellten Stromwert nicht, so wird in einem dritten Schritt abgefragt, ob die momentane Durchflussmenge einen ersten voreingestellten Durchfluss-Grenzwert überschreitet, sich die Pumpe also im gefährdeten Betrieb befindet. Ist dies nicht der Fall, so wird die Pumpe wieder im "normalen" Betrieb weiter betrieben, der Pumpenmotor also zur Erreichung der eingestellten Fördermenge betrieben. Wird jedoch der voreingestellte Durchfluss-Grenzwert überschritten, befindet sich also die Pumpe im gefährdeten Betrieb, so wird die Durchflussmenge auf einen Durchfluss-Sicherheitswert reduziert, die Spülpumpe also in einen sicheren Betriebszustand gesetzt. Nun wird die Pumpe wieder in den "normalen" Betrieb geschaltet, in welchem der Motor zur Erreichung der eingestellten Förderleistung angesteuert wird. Dann beginnt das gespeicherte Steuerprogramm wieder mit dem ersten Schritt.

Um einen sicheren und auch von schnellen, unkontrollierten Schwingungen freien Betrieb zu gewährleisten, wird die in Fig. 3 aufgezeigte Timer-Interrupt-Service-Routine gefahren. Hierbei wird wieder abgefragt, ob sich die Pumpe im gefährdeten Betrieb befindet, also der Durchfluss-Grenzwert überschritten wurde. Danach wird abgefragt, ob zuvor ein Fehlerfall aufgetreten ist, die Durchflussmenge also zuvor auf den Durchfluss-Sicherheitswert reduziert wurde. Ist dies der Fall, so wird abgefragt, ob das ursprüngliche Leistungsniveau entsprechend der voreingestellten Durchflussmenge erreicht wurde. Ist dies nicht der Fall, so wird die Leistung der Pumpe (entsprechend dem Motorstrom) erhöht und die Pumpe wird wieder in der Timer-Interrupt-Service-Routine gemäß Fig. 3 mit der ersten Abfrage weiter betrieben. Wurde das Leistungsniveau erreicht, so wird die Pumpe ohne Erhöhung der Leistung in dieser Routine weiter betrieben. Diese Routine wird zeitgesteuert abgefahren, so dass keine abrupte, zu schnellen Regelschwingungen führende Leistungssteigerung stattfindet, sondern eine Zweipunkt-Regelung mit einer festgelegten Schaltperiode stattfindet.

## Patentansprüche

1. Spüleinrichtung insbesondere für die Endoskopie, umfassend eine elektrisch angetriebene Pumpe (10), insbesondere eine Peristaltik-Rollenpumpe,
eine Steuerungseinrichtung (20) zum Feststellen und/oder Einstellen einer Durchflussmenge, und
eine Strom-Messeinrichtung (11) zum Feststellen eines von der Pumpe (10) aufgenommenen Stroms **dadurch gekennzeichnet, dass**, die Steuerungseinrichtung (20) derart ausgebildet ist, dass dann, wenn die Durchflussmenge einen voreingestellten Durchfluss-Grenzwert überschreitet und gleichzeitig der Strom einen ersten voreingestellten Stromwert überschreitet, die Durchflussmenge auf einen Durchfluss-Sicherheitswert reduziert wird.

2. Spüleinrichtung nach Anspruch 1,
**dadurch gekennzeichnet, dass**
der Durchfluss-Sicherheitswert gleich dem Durchfluss-Grenzwert ist.

3. Spüleinrichtung nach einem der vorhergehenden Ansprüche,
**dadurch gekennzeichnet, dass**
die Steuerungseinrichtung (20) derart ausgebildet ist, dass dann, wenn der Strom einen zweiten voreingestellten Stromwert überschreitet, der höher ist als der erste Stromwert die Pumpe (10) abgeschaltet und/oder ein Warnsignal erzeugt wird.

4. Spüleinrichtung nach einem der vorhergehenden Ansprüche,
**dadurch gekennzeichnet, dass**
nach Reduzieren der Durchflussmenge auf den Durchfluss-Sicherheitswert die Durchflussmenge mit einer voreingestellten Steigerungsrate bis zu einer voreingestellten Soll-Durchflussmenge erhöht wird.

5. Verfahren zum Betreiben einer Spülpumpe, insbesondere für die Endoskopie, die eine elektrisch angetriebene Pumpe, insbesondere eine Peristaltik-Rollenpumpe aufweist,
eine Durchflussmenge festgestellt und/oder eingestellt wird,
ein Strom festgestellt wird, welcher von der Pumpe aufgenommen wird, **dadurch gekennzeichnet, dass** dann, wenn die Durchflussmenge einer voreingestellten Durchfluss-Grenzwert überschreitet und gleichzeitig der Strom einen ersten voreingestellten Stromwert überschreitet, die Durchflussmenge auf einen Durchfluss-Sicherheitswert reduziert wird.

6. Verfahren nach Anspruch 5,
**dadurch gekennzeichnet, dass**
der Durchfluss-Sicherheitswert gleich dem Durchfluss-Grenzwert ist.

7. Verfahren nach einem der Ansprüche 5 oder 6,
**dadurch gekennzeichnet, dass**
dann, wenn der Strom einen zweiten voreingestellten Stromwert überschreitet, der höher ist als der erste Stromwert, die Pumpe abgeschaltet und/oder ein Warnsignal erzeugt wird.

8. Verfahren nach einem der Ansprüche 5 - 7,
**dadurch gekennzeichnet, dass**
nach einem Reduzieren der Durchflussmenge auf den Durchfluss-Sicherheitswert die Durchflussmenge mit einer voreingestellten Steigerungsrate bis zu einer voreingestellten Soll-Durchflussmenge erhöht wird.

## Claims

1. Irrigation device, especially for endoscopy, comprising an electrically driven pump (10), especially a peristaltic roller pump,
a control device (20) for ascertaining and/or adjusting the through-flow rate, and a current-measuring device (11) for ascertaining the current taken by the pump (10),
**characterised in that**
the control device (20) is so arranged that when the through-flow rate exceeds a pre-set through-flow limit value and at the same time the current exceeds a first pre-set value of current, the through-flow rate is reduced to a through-flow safety value.

2. Irrigation device according to claim 1,
**characterised in that**
the through-flow safety value is equal to the through-flow limit value.

3. Irrigation device according to one of the preceding claims,
**characterised in that**
the control device (20) is so arranged that when the current exceeds a second pre-set value of current which is higher than the first value of current, the pump (10) is switched off and/or a warning signal is produced.

4. Irrigation device according to one of the preceding claims,
**characterised in that**
after reduction of the through-flow rate to the through-flow safety value the through-flow rate is increased at a pre-set rate of increase to a pre-set desired through-flow rate.

5. Method of operating an irrigation pump, especially for endoscopy, comprising an electrically driven pump, especially a peristaltic roller pump,
the through-flow rate being ascertained and/or adjusted,
the current which is taken by the pump being ascertained,
**characterised in that**
when the through-flow rate exceeds a pre-set through-flow limit value and at the same time the current exceeds a first pre-set value of current, the through-flow rate is reduced to a through-flow safety value.

6. Method according to claim 5,
**characterised in that**
the through-flow safety value is equal to the through-flow limit value.

7. Method according to one of claims 5 or 6,
**characterised in that**
when the current exceeds a second pre-set value of current which is higher than the first value of current, the pump is switched off and/or a warning signal is produced.

8. Method according to one of claims 5 - 7,
**characterised in that**
after reduction of the through-flow rate to the through-flow safety value the through-flow rate is increased at a pre-set rate of increase to a pre-set desired through-flow rate.

## Revendications

1. Dispositif de nettoyage, en particulier pour l'endoscopie, comportant une pompe (10) à entraînement électrique, en particulier une pompe à rouleaux péristaltique, un dispositif de commande (20) destiné à détecter et/ou à régler un débit, et un dispositif de mesure du courant (11) destiné à détecter un courant reçu par la pompe (10),
**caractérisé en ce que** le dispositif de commande (20) est configuré de telle sorte que, lorsque le débit est supérieur à une valeur limite préréglée du débit et que, en même temps, le courant est supérieur à une première valeur préréglée du courant, le débit est diminué à une valeur de sécurité.

2. Dispositif de nettoyage selon la revendication 1, **caractérisé en ce que** la valeur de sécurité du débit est égale à la valeur limite du débit.

3. Dispositif de nettoyage selon l'une quelconque des revendications précédentes, **caractérisé en ce que** le dispositif de commande (20) est configuré de telle sorte que, lorsque le courant est supérieur à une deuxième valeur préréglée du courant, laquelle est supérieure à la première valeur du courant, la pompe (10) est débranchée et/ou un signal d'alarme est délivré.

4. Dispositif de nettoyage selon l'une quelconque des revendications précédentes, **caractérisé en ce qu'**après la diminution du débit jusqu'à la valeur de sécurité, le débit est augmenté avec un taux d'accroissement préréglé jusqu'à un débit de consigne préréglé.

5. Procédé pour le fonctionnement d'une pompe de nettoyage, en particulier pour l'endoscopie, qui comporte une pompe à entraînement électrique, en particulier une pompe à rouleaux péristaltique, dans lequel procédé un débit est détecté et/ou réglé, un courant, qui est reçu par la pompe, est détecté,
**caractérisé en ce que**, lorsque le débit est supérieur à une valeur limite préréglée du débit et que, en même temps, le courant est supérieur à une première valeur préréglée du courant, le débit est diminué à une valeur de sécurité.

6. Procédé selon la revendication 5, **caractérisé en ce que** la valeur de sécurité du débit est égale à la valeur limite du débit.

7. Procédé selon la revendication 5 ou 6, **caractérisé en ce que**, lorsque le courant est supérieur à une deuxième valeur préréglée du courant, laquelle est supérieure à la première valeur du courant, la pompe est débranchée et/ou un signal d'alarme est délivré.

8. Procédé selon l'une quelconque des revendications 5 à 7, **caractérisé en ce qu'**après une diminution du débit jusqu'à la valeur de sécurité, le débit est augmenté avec un taux d'accroissement préréglé jusqu'à un débit de consigne préréglé.
